(19) Europäisches Patentamt

European Patent Office

Office européen des brevets

(11) **EP 2 500 456 A1**

(12) # EUROPEAN PATENT APPLICATION
published in accordance with Art. 153(4) EPC

(43) Date of publication:
**19.09.2012 Bulletin 2012/38**

(21) Application number: **10830058.3**

(22) Date of filing: **10.11.2010**

(51) Int Cl.:
*D04H 3/00* (2012.01)   *A61L 15/16* (2006.01)
*A61L 27/00* (2006.01)   *D01D 5/04* (2006.01)
*D01F 6/00* (2006.01)   *D04H 1/40* (2012.01)
*D04H 1/72* (2012.01)

(86) International application number:
**PCT/JP2010/070415**

(87) International publication number:
**WO 2011/059102 (19.05.2011 Gazette 2011/20)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **11.11.2009 JP 2009257978**

(71) Applicant: **Teijin Limited
Osaka-shi, Osaka 541-0054 (JP)**

(72) Inventors:
• **HONDA, Susumu
Hino-shi
Tokyo 191-0065 (JP)**

• **KAGEYAMA, Yukako
Hino-shi
Tokyo 191-0065 (JP)**
• **SATAKE, Makoto
Hino-shi
Tokyo 191-0065 (JP)**
• **KANEKO, Hiroaki
Hino-shi
Tokyo 191-0065 (JP)**

(74) Representative: **Hallybone, Huw George
Carpmaels & Ransford
One Southampton Row
London
WC1B 5HA (GB)**

(54) **MOLDED FIBER ARTICLE**

(57)    Disclosed is a fibrous formed article having excellent hydrophilicity, which contains a hydrophobic polymer and amphiphilic molecules and has an average fiber diameter of 0.05 to 50 $\mu$m, the amphiphilic molecules segregating to the fiber surface.

EP 2 500 456 A1

**Description**

Technical Field

The present invention relates to a fibrous formed article containing a hydrophobic polymer and amphiphilic molecules, the amphiphilic molecules segregating to the fiber surface.

Background Art

In recent years, as a treatment for severely damaged or lost biological tissues and organs, intensive studies have been made on regenerative medicine for the reconstruction of the original biological tissues or organs utilizing the differentiation and proliferation potentials of cells. When cells differentiate or proliferate in vivo, an extracellular matrix functions as a scaffold for tissue construction. However, in the case where the tissue has been severely damaged or lost, it is necessary to compensate with an artificial or natural material until the cells themselves produce a matrix. That is, a scaffold material is an important factor that provides an optimal environment for tissue construction. Characteristics required from such scaffold materials include bioabsorbability, adhesion to cells or proteins, porosity, and dynamic strength. For the purpose of creating materials that satisfy these characteristics, studies have been made on synthetic polymers (polyglycolic acid, polylactic acid, polycaprolactone, etc.), natural polymers (collagen, gelatin, elastin, hyaluronic acid, alginic acid, chitosan, etc.), inorganic materials (hydroxyapatite, β-tricalcium phosphate), and composites thereof.

Among synthetic polymers, formed bodies made of fibers processed from an aliphatic polyester have been used in various applications including sutures, bioabsorbable sheets, and the like. In addition, nanofibers produced by electrostatic spinning or the like have a large surface area and thus have high adhesion to cells. Therefore, their applications to cell culture carriers or scaffold materials for regenerative medicine have been studied.

As mentioned above, adhesion is one of the important characteristics required from a scaffold material. However, aliphatic polyesters, particularly polylactic acid and copolymers of polylactic acid and polyglycolic acid, are hydrophobic, and thus have a problem in that when they are used in a hydrophilic environment, their interactions with cells or proteins are limited.

In order to solve this problem, methods for imparting hydrophilicity to such hydrophobic polymers have been studied, including a method in which a hydrophilic polymer such as polyethylene glycol is blended and a method in which a hydrophilic polymer is introduced into the polymer main chain as a block copolymer (e.g., K. Kim, M. Yu, Biomaterials., 24, 4977 (2003), N. Saito, T. Okada, Nat. Biotech. , 19, 332 (2001)). However, in order to impart hydrophilicity, a relatively large amount of a hydrophilic polymer is necessary. In addition, in the case of a block copolymer, the molecular weight is insufficient, and satisfactory dynamic strength has not yet been achieved.

A porous fiber made of a polymer soluble in a hydrophobic solvent and an organic compound having a plurality of hydroxy groups has also been disclosed (WO 2004/072336, Description), and it is stated that the porous fiber is useful as a cell culture substrate.

However, the porous fiber is a structure that is advantageous for delivering nutrients and the like to cultured cells, and there is no description about the hydrophilicity or surface composition of a fiber structure.

Further, an aliphatic polyester nanofiber containing a phospholipid is also known (WO 2006/022430, Description). However, this invention relates to a fiber structure that serves as a substrate suitable for cell culture, which is characterized by having pores in the fiber surface. There is no disclosure about a nanofiber having a phospholipid or like amphiphilic molecules segregating to the fiber surface.

Disclosure of the Invention

A problem to be solved by the present invention is to provide a hydrophobic polymer fibrous formed article having required hydrophilicity even when the amount of amphiphilic molecules contained therein is small.

The present inventors conducted extensive research to solve the problems mentioned above and, as a result, found the following. In a fibrous formed article containing a hydrophobic polymer and amphiphilic molecules produced under specific conditions, the amphiphilic molecules segregate to the fiber surface. As a result, hydrophilicity can be imparted even when the amount of amphiphilic molecules added is small. In addition, original characteristics of the hydrophobic polymer, such as mechanical properties, are not impaired. They thus accomplished the invention.

That is, the invention is a fibrous formed article containing a hydrophobic polymer and amphiphilic molecules. The fibrous formed article has an average fiber diameter of 0.05 to 50 μm, and the amphiphilic molecules segregate to the fiber surface.

Best Mode for Carrying Out the Invention

In the invention, a fibrous formed article means a three-dimensional formed body produced by stacking, weaving, knitting, or otherwise processing one or several fibers obtained. Specifically, the fibrous formed article may be in the form of a nonwoven fabric, for example. Further, tubes, meshes, and the like processed therefrom are also encompassed by "fibrous formed article" used herein, and they are suitable for use in the field of regenerative medicine.

The fibrous formed article of the invention has an average fiber diameter of 0. 05 to 50 $\mu$m. When the average fiber diameter is less than 0.05 $\mu$m, the strength of the fibrous formed article cannot be maintained; therefore, this is undesirable. When the average fiber diameter is more than 50 $\mu$m, such fibers have a small specific surface area, whereby the number of cells adhering thereto is reduced; therefore, this is undesirable. The average fiber diameter is still more preferably 0.2 to 20 $\mu$m. Incidentally, a fiber diameter is the diameter of a fiber cross-section. The shape of a fiber cross-section is not limited to circular, and may also be elliptical or modified. In this case, the average of the lengths of the major axis and minor axis of the elliptical shape is calculated as the fiber diameter. In addition, when the fiber cross-section is not either circular or elliptical, the shape is approximated to a circle or ellipse to calculate the fiber diameter.

In the invention, examples of hydrophobic polymers include aliphatic polyesters such as polylactic acid, polylactic acid-polyglycolic acid copolymers, and polycaprolactone, as well as polycarbonate, polystyrene, polyarylate, polymethyl methacrylate, polyethyl methacrylate, cellulose diacetate, cellulose triacetate, polyvinyl acetate, polyvinyl methyl ether, polyethylene succinate, and copolymers thereof. Mixtures of two or more kinds thereof are also included. Among them, aliphatic polyesters, polycarbonate, polystyrene, and polyarylate are preferable.

It is preferable that the aliphatic polyester used in the invention is a bioabsorbable polymer. Examples of bioabsorbable polymers include polylactic acid, polyglycolic acid, polylactic acid-polyglycolic acid copolymers, polycaprolactone, polyglycerol sebacate, polyhydroxyalkanoates, polybutylene succinate, and derivatives thereof.

Among them, it is preferable that the polymer is at least one member selected from the group consisting of polyglycolic acid, polylactic acid, polycaprolactone, and copolymers thereof. Polylactic acid and polylactic acid-glycolic acid copolymers are most preferable.

At this time, polylactic acid copolymers having a smaller amount of monomer components that impart stretchability are more desirable. Examples of monomer components that impart stretchability include soft components such as caprolactone monomers, ethylene glycol, 1,2-propylene glycol, 1,3-propylene glycol, 1,2-butanediol, 1,4-butanediol, polycaprolactone diol, polyalkylene carbonate diols, and polyethylene glycol units. It is preferable that the amount of soft components is less than 20% based on the weight of the polymer. When the amount of soft components is larger, the fibrous formed article tends to be not self-supporting, resulting in a fibrous formed article that is too soft and difficult to handle.

Polymer-forming monomers in polylactic acid include L-lactic acid and D-lactic acid, but there are no particular limitations. In addition, there are no particular limitations on the optical purity or molecular weight of the polymer, the proportions of L- and D-forms, or their arrangement. However, a polymer having a higher proportion of L-form is preferable. It is also possible to use a stereocomplex of poly(L-lactic acid) and poly(D-lactic acid).

The molecular weight of the polymer is $1 \times 10^3$ to $5 \times 10^6$, preferably $1 \times 10^4$ to $1 \times 10^6$, and more preferably $5 \times 10^4$ to $5 \times 10^5$. In addition, the terminal structure of the polymer and a catalyst for polymerization can be arbitrarily selected.

In the fibrous formed article of the invention, as long as the object of the invention is not impaired, it is also possible to use other polymers or other compounds together. Examples thereof include polymer copolymerization, polymer blending, and compound mixing.

It is preferable that the polymer has high purity. It particular, with respect to residues contained in the polymer, such as additives, plasticizers, residual catalysts, residual monomers, and residual solvents used in forming or post-processing, the smaller the amount of residues the better. Particularly in the case of medical applications, the amount needs to be controlled below the safety standard.

The fibrous formed article of the invention is a fibrous formed article containing amphiphilic molecules in an amount of 0.01 to 20 wt% based on the weight of the polymer. When the amphiphilic molecule content is less than 0.01 wt%, no hydrophilicity is exhibited, while when the content is more than 20 wt%, the durability of the fibrous formed article itself decreases; therefore, this is undesirable. The content is preferably 0.02 to 15 wt%, and still more preferably 0.05 to 10 wt%.

It is preferable that the amphiphilic molecules in the invention are at least one member selected from the group consisting of phospholipids, sorbitan fatty acid esters, glycolipids, steroids, and polyamino acids. Specific examples of amphiphilic molecules include phospholipids such as phosphatidylcholine, phosphatidylethanolamine, phosphatidylserine, and phosphatidylglycerol; sorbitan fatty acid esters such as sorbitan monolaurate, sorbitan monopalmitate, sorbitan monostearate, sorbitan monooleate, sorbitan sesquioleate, and sorbitan trioleate; glycolipids such as galactocerebroside, glucocerebroside, globoside, lactosylceramide, trihexosylceramide, paragloboside, galactosyldiacylglycerol, sulfoquinovosyldiacylglycerol, phosphatidylinositol, and glycosylpolyprenol phosphate; steroids such as cholesterol, cholic acid, sapogenin, and digitoxin; and polyamino acids such as polyaspartic acid, polyglutamic acid, and polylysine.

In the invention, it is preferable that the amphiphilic molecules are dried before use by lyophilization, hot-air drying, vacuum drying, etc.

In the fibrous formed article of the invention, amphiphilic molecules segregate to the fiber surface of the fibrous formed article. Segregation to the fiber surface means that in the fibrous formed article containing a hydrophobic polymer and amphiphilic molecules, the proportion of amphiphilic molecules in the fiber surface is higher than the proportion of amphiphilic molecules in other parts of the fibrous formed article.

Examples of techniques for evaluating the distribution of amphiphilic molecules in the fibrous formed article of the invention include, but are not limited to, X-ray photoelectron spectroscopy (abbreviated as XPS or ESCA (Electron Spectroscopy for Chemical Analysis)), a time-of-flight secondary ion mass spectrometer (TOF-SIMS), and a transmission electron microscope (TEM).

In the examples and comparative examples described herein, ESCA was employed as a technique for analyzing the surface composition of a fibrous formed article. From the value of the number of carbon atoms C (at%) and the value of the number of oxygen atoms O (at%) determined by ESCA measurement on a fibrous formed article, the distribution of amphiphilic molecules in the fiber surface can be calculated as a weight fraction. However, hydrogen atoms cannot be measured by ESCA measurement. Therefore, the number of atoms other than hydrogen is calculated.

More specifically, for example, the number of carbon atoms C (at%) in a composition containing a hydrophobic polymer and amphiphilic molecules is represented by the following equation:

```
(number of carbon atoms C (at%) of a composition

containing a hydrophobic polymer and amphiphilic molecules)

= {(mol% of the amphiphilic molecules) × (number of

carbon atoms in 1 mol of the amphiphilic molecules) + (mol%

of monomer units in the hydrophobic polymer) × (number of carbon

atoms in 1 mol of monomer units in the hydrophobic polymer)}

÷ {(mol% of the amphiphilic molecules) × (number of atoms

other than hydrogen in 1 mol of the amphiphilic molecules) +

(mol% of hydrophobic polymer units) × (number of atoms other

than hydrogen in 1 mol of hydrophobic polymer units}.
```

Therefore, by substituting the value of the number of carbon atoms C (at%) determined by ESCA measurement into the above equation, the mol% of each component in the fiber surface and also the weight fraction can be calculated. Likewise, calculation from the value of the number of oxygen atoms O (at%) is also possible. In this description, the average of values calculated from the value of the number of carbon atoms C (at%) and the value of the number of oxygen atoms O (at%) was taken as the weight fraction of amphiphilic molecules in the surface of the fibrous formed article. Then, the obtained average was compared with the weight fraction of amphiphilic molecules contained in the fibrous formed article to evaluate the degree of surface segregation.

Here, "surface segregation degree of amphiphilic molecules", which represents the degree of the surface segregation of amphiphilic molecules, is defined as in the following equation:

$$\text{(surface segregation degree of amphiphilic molecules)}$$

$$= \text{(weight fraction of amphiphilic molecules in the surface of}$$

$$\text{a fibrous formed article)} / \text{(weight fraction of amphiphilic}$$

$$\text{molecules contained in the fibrous formed article).}$$

In the fibrous formed article of the invention, it is preferable that the value is 5.0 or more. In the case where the value is less than 5, the hydrophilicity of the fibrous formed article may be insufficient.

Incidentally, "surface of a fibrous formed article" herein means the region to be measured by the above analysis method and refers, for example, to a region that is 10 nm deep from the outermost surface. In addition, in the expression "a fibrous formed article with amphiphilic molecules segregating to the fiber surface" in the invention, "to segregate to the surface" is not limited to segregation only to this region; when amphiphilic molecules segregate to a deeper region from the outermost surface, it is also encompassed thereby (the degree is not limited).

The fibrous formed article of the invention may further contain a third component in addition to a hydrophobic polymer and amphiphilic molecules. Examples of such components include cell growth factors such as FGF (fibroblast growth factor), EGF (epidermal growth factor), PDGF (platelet derived growth factor), TGF-β (transforming growth factor-β), NGF (nerve growth factor), HGF (hepatocyte growth factor), and BMP (bone morphogenetic protein).

The entire thickness of the fibrous formed article is not particularly limited, and is preferably 25 $\mu$m to 200 $\mu$m, and still more preferably 50 to 100 $\mu$m.

It is preferable that the fibrous formed article of the invention is formed of a filament. "Filament" refers to a fibrous formed article that is produced without performing a fiber-cutting step during the steps from spinning to processing into a fibrous formed article, and it is preferable that the production is performed by electrostatic spinning.

Electrostatic spinning is a method in which a high voltage is applied to a solution of a polymer dissolved in a solvent, thereby producing a fibrous formed article on the electrode. Electrostatic spinning includes the following steps: a step of dissolving a polymer in a solvent to produce a solution; a step of applying a high voltage to the solution; a step of ejecting the solution; a step of evaporating the solvent from the ejected solution to produce a fibrous formed article; an optional step of dissipating charges on the produced fibrous formed article; and an optional step of accumulating the fibrous formed article by charge dissipation.

The following describes the stage of dissolving a polymer in a solvent to produce a solution in electrostatic spinning. In the production method of the invention, it is preferable that the concentration of the hydrophobic polymer in the solution relative to the solvent is 1 to 30 wt%. When the concentration of the hydrophobic polymer is less than 1 wt%, the concentration is so low that it is difficult to produce a fibrous formed article; therefore, this is undesirable. When the concentration is more than 30 wt%, the resulting fibrous formed article has a large fiber diameter; therefore, this is undesirable. It is more preferable that the concentration of the hydrophobic polymer in the solution relative to the solvent is 2 to 20 wt%.

One kind of solvent may be used alone, and it is also possible to use two or more kinds of solvents in combination. The solvent is not particularly limited as long as it is capable of dissolving a hydrophobic polymer and amphiphilic molecules and can evaporate during spinning to produce fibers. Examples thereof include acetone, chloroform, ethanol, 2-propanol, methanol, toluene, tetrahydrofuran, benzene, benzyl alcohol, 1,4-dioxane, 1-propanol, dichloromethane, carbon tetrachloride, cyclohexane, cyclohexanone, phenol, pyridine, trichloroethane, acetic acid, formic acid, hexafluoro-2-propanol, hexafluoroacetone, N,N-dimethylformamide, N,N-dimethylacetamide, acetonitrile, N-methyl-2-pyrrolidinone, N-methylmorpholine-N-oxide, 1,3-dioxolane, methyl ethyl ketone, and mixed solvents of the above solvents.

In the case where the fibrous formed article of the invention is produced by electrostatic spinning, it is preferable to control moisture because water affects the fiber surface. In order to control moisture, it is preferable that the solvent is dry. Specifically, it is preferably controlled to 2,000 ppm or less, and more preferably 1,000 ppm or less. The method for drying a solvent may be, but is not particularly limited to, drying by distillation over a desiccant.

Meanwhile, in the case where the solvent contains water, it is preferable that a water-miscible solvent is contained. In the cases where no water-miscible solvent is contained, the segregation of amphiphilic molecules to the fiber surface is insufficient; therefore, this is undesirable.

Among these, in terms of handleability, physical properties, etc., dichloromethane and ethanol are preferable.

Next, the following describes the stage of applying a high voltage to the solution, the stage of ejecting the solution, and the stage of evaporating the solvent from the ejected solution to produce a fibrous formed article.

In the production method for the fibrous formed article of the invention, in order to eject a solution of a hydrophobic polymer and amphiphilic molecules to produce a fibrous formed article, it is necessary to apply a high voltage to the solution. The method of voltage application is not particularly limited as long as the solution of a hydrophobic polymer is ejected to produce a fibrous formed article. Examples thereof include a method in which an electrode is inserted into the solution to apply a voltage and a method in which a voltage is applied to a solution-ejecting nozzle.

In addition, separately from the electrode for applying a voltage to the solution, an auxiliary electrode may also be provided. The voltage to be applied is not particularly limited as long as the fibrous formed article is produced, and a voltage within a range of 5 to 50 kV is usually preferable. In the case where the applied voltage is less than 5 kV, the solution is not ejected, and a fibrous formed article is not produced; therefore, this is undesirable. In the case where the applied voltage is more than 50 kV, the electrode is discharged to the ground electrode; therefore, this is undesirable. It is more preferable that the voltage is within a range of 5 to 30 kV. A desired electrical potential may be created by any known suitable method.

As a result, immediately after the solution of a hydrophobic polymer and amphiphilic molecules is ejected, the solvent used for dissolution is volatilized, whereby a fibrous formed article is produced. Spinning is usually performed in atmospheric air at room temperature. However, in the case where volatilization is insufficient, it may also be performed under a negative pressure or in a high-temperature atmosphere. The spinning temperature depends on the evaporation behavior of the solvent and the viscosity of the spinning solution, and is usually within a range of 0 to 50°C. In the case where a fibrous formed article is produced by electrostatic spinning, water affects the fiber surface. Accordingly, in order to obtain a fiber with a smooth surface having amphiphilic molecules segregating thereto, it is preferable that spinning is performed in a low-humidity atmosphere. Specifically, the relative humidity is 35% or less, preferably 30% or less, more preferably 25% or less, and particularly preferably 20% or less.

Next, the following describes the stage of dissipating charges on the produced fibrous formed article. The method for dissipating charges on the fibrous formed article is not particularly limited. A preferred example is a method in which charges are dissipated by an ionizer. An ionizer is an apparatus that generates ions from an internal ion generator and releases the ions to a charged object to dissipate charges on the charged object. A preferred example of an ion generator that forms the ionizer used in the production method for the fibrous formed article of the invention is an apparatus that generates ions by applying a high voltage to an internal discharge needle.

Next, the following describes the stage of accumulating the fibrous formed article by the charge dissipation mentioned above. The method for accumulating a fibrous formed article by charge dissipation is not particularly limited. According to a usual method, static electricity on the fibrous formed article is removed by charge dissipation to allow the fibrous formed article to fall and accumulate due to its own weight. In addition, as neccessary, it is also possible to perform a method in which the fibrous formed article from which static electricity has been dissipated is sucked and accumulated on a mesh or a method in which air convection is caused in the apparatus to accumulate the fibrous formed article on a mesh.

As long as the object of the inveniton is not impaired, it is possible to optionally perform processes in which a flocculent fiber structure is further stacked on the surface of the fibrous formed article of the invention, a flocculent structure is inserted into the fibrous formed article of the invention to form a sandwich structure, etc.

In medical applications, it is also possible to optionally perform a coating treatment to further impart antithrombogenicity or coat the surface with an antibody or a physiologically active substance. At this time, the coating method, treatment conditions, and chemicals used for the treatment may be arbitrarily selected as long as the fiber structure is not extremely destroyed and the object of the invention is not impaired.

The fibrous formed article of the invention may also contain a medical agent inside the fiber. In the case where the formation is performed by electrostatic spinning, medical agents to be used are not particularly limited as long as they are soluble in a volatile solvent and their physiological activities are not lost by dissolution.

Specific examples of such medical agents include tacrolimus and analogs thereof, statin drugs, and taxane anticancer drugs.

The medical agents may also be protein preparations and nucleic acid medicines as long as their activities can be maintained in a volatile solvent. In addition, non-medical agents may also be contained, examples thereof including metals, polysaccharides, fatty acids, surfactants, and volatile-solvent-resistant microorganisms.


Examples

First, measurement methods employed in the examples and comparative examples are summarized.


1. Average Fiber Diameter:

The surface of an obtained fibrous formed article was photographed with a scanning electron microscope (Keyence Corporation: trade name "VE 8800") at a magnification of $2,000\times$. In the obtained photograph, 20 points were selected at random to measure the fiber diameter. The average of all the fiber diameters was calculated as the average fiber diameter. The number of samples is 20.

2. Average Thickness:

Using a high-accuracy digital measuring instrument (Mitutoyo Corporation: trade name "Litematic VL-50"), the thickness of a fibrous formed article was measured with a measuring force of 0.01 N, and the average of ten samples was calculated. Incidentally, this measurement was performed with a minimum measuring force required to use the measuring instrument.

3. Value of the Number of Carbon Atoms C (at%) and Value of the Number of Oxygen Atoms O (at%) in Fiber Surface:

Detection was performed using ESCALAB 200 manufactured by VG as a photoelectron spectrometer and MgKa rays as X-rays (1253.6 eV) at a photoelectron takeoff angle of 45°.

4. Hydrophilicity Test:

An obtained fibrous formed article was inserted into a filter holder ($\phi$ 8 mm) using a silicon sheet (1 mm) as a gasket. Subsequently, the filter holder was placed below a cylinder tube made of glass, and a 7% albumin (from bovine serum, pH 5.2: manufactured by Wako Pure Chemical Industries) /PBS (20012 Phosphate-Buffered Saline, liquid: manufactured by GIBCO) liquid was poured at a rate of 3.87 mL/min. When the 7% albumin /PBS solution reaches a certain amount, the liquid permeates the sheet. Wettability was determined from the height of the liquid column required for the permeation. That is, a lower height of the liquid column indicates better hydrophilicity. The test was performed using three samples, and their average was used.

[Example 1]

0.1 part by weight of lyophilized dioleoylphosphatidylethanolamine (manufactured by NOF Corporation) and 9.9 parts by weight of polylactic acid (weight average molecular weight: 262,000, manufactured by Purac) were dissolved in 90 parts by weight of dichloromethane (moisture content by Karl Fischer: 500 ppm or less) dried over a molecular sieve (manufactured by Union Showa, Pellet 3A) to prepare a homogeneous solution. Spinning was performed by electrostatic spinning at a humidity of 25% or less to give a sheet-like fibrous formed article. The inner diameter of the ejection nozzle was 0.8 mm, the voltage was 8 kV, and the distance from the ejection nozzle to a flat plate was 25 cm. The flat plate served as the cathode in spinning. The obtained fibrous formed article had an average fiber diameter of 4.5 $\mu$m and a thickness of 104 $\mu$m. The number of carbon atoms C (at%) and the number of oxygen atoms O (at%) in the fiber surface were 62.4 and 37.3, respectively. The weight percentage of dioleoylphosphatidylethanolamine in the fiber surface was 11.5 wt%. The surface segregation degree of amphiphilic molecules is 11.5.

[Comparative Example 1]

A fibrous formed article was prepared in the same manner as in Example 1, except that dioleoylphosphatidylethanolamine (manufactured by NOF Corporation) was not contained. The fibrous formed article forming the obtained fiber structure had an average diameter of 5.4 $\mu$m and a thickness of 95 $\mu$m.

[Comparative Example 2]

0.1 part by weight of non-lyophilized dioleoylphosphatidylethanolamine (manufactured by NOF Corporation) and 9.9 parts by weight of polylactic acid (weight average molecular weight: 262, 000, manufactured by Purac) were dissolved in 90 parts by weight of dichloromethane (moisture content by Karl Fischer: more than 2, 000 ppm), and a fibrous formed article was prepared at a humidity of 42 to 55%. The obtained fibrous formed article had an average fiber diameter of 4.1 $\mu$m and a thickness of 98 $\mu$m, and the fiber surface had a porous structure. The number of carbon atoms C (at%) and the number of oxygen atoms O (at%) in the fiber surface were 60.4 and 39.5, respectively. The amount of dioleoylphosphatidylethanolamine in the fiber surface was 2.0 wt%. The surface segregation degree of amphiphilic molecules was 2.0, indicating that the segregation of amphiphilic molecules to the fiber surface was insufficient.

[Example 2]

A fibrous formed article was prepared in the same manner as in Example 1, except that dioleoylphosphatidylethanolamine (manufactured by NOF Corporation) was replaced with dilauroylphosphatidylcholine (manufactured by NOF Corporation). The obtained fibrous formed article had an average fiber diameter of 4.3 $\mu$m and a thickness of 102 $\mu$m. The number of carbon atoms C (at%) and the number of oxygen atoms O (at%) in the fiber surface were 62.1 and 37.2, respectively. The amount of dilauroylphosphatidylcholine in the fiber surface was 13.4 wt%. The surface segregation degree of amphiphilic molecules is 13.4.

[Example 3]

The same procedure as in Example 1 was performed, except that polycaprolactone (average molecular weight: about 70,000 to 100, 000, manufactured by Wako Pure Chemical Industries) was used in place of polylactic acid. The obtained fibrous formed article had an average fiber diameter of 4.5 $\mu$m and a thickness of 99 $\mu$m. The number of carbon atoms C (at%) and the number of oxygen atoms O (at%) in the fiber surface were 75.5 and 24.1, respectively. The amount of dioleoylphosphatidylethanolamine in the fiber surface was 9.7 wt%. The surface segregation degree of amphiphilic molecules is 9.7.

[Example 4]

The same procedure as in Example 1 was performed, except that 1 part by weight of polycarbonate (manufactured by Teij in Chemicals: trade name "Panlite L-1250") was used in place of polylactic acid. The average fiber diameter was 3.2 $\mu$m, and the thickness was 102 $\mu$m. The number of carbon atoms C (at%) and the number of oxygen atoms O (at%) in the fiber surface were 83.85 and 15.78, respectively. The amount of dioleoylphosphatidylethanolamine in the fiber surface was 10.1 wt%. The surface segregation degree of amphiphilic molecules is 10.1.

[Example 5]

The same procedure as in Example 1 was performed, except that polystyrene (average molecular weight: 250,000, manufactured by Kanto Chemical) was used in place of polylactic acid. The average fiber diameter was 6.1 $\mu$m, and the thickness was 102 $\mu$m. The number of carbon atoms C (at%) and the number of oxygen atoms O (at%) in the fiber surface were 98.3 and 1.4, respectively. The amount of dioleoylphosphatidylethanolamine in the fiber surface was 9.8 wt%. The surface segregation degree of amphiphilic molecules is 9.8.

[Example 6]

The same procedure as in Example 1 was performed, except that polyarylate (manufactured by Unitika: trade name "U-Polymer U-100") was used in place of polylactic acid. The average fiber diameter was 3.4 $\mu$m, and the thickness was 105 $\mu$m. The number of carbon atoms C (at%) and the number of oxygen atoms O (at%) in the fiber surface were 84.7 and 14.9, respectively. The amount of dioleoylphosphatidylethanolamine in the fiber surface was 10.8 wt%. The surface segregation degree of amphiphilic molecules is 10.8.

[Example 7]

A fibrous formed article was prepared in the same manner as in Example 1, except that 0.5 part by weight of lyophilized dioleoylphosphatidylethanolamine (manufactured by NOF Corporation) and 9.5 parts by weight of polylactic acid (weight average molecular weight: 262,000, manufactured by Purac) were dissolved in 90 parts by weight of dichloromethane (moisture content by Karl Fischer: 500 ppm or less) dried over a molecular sieve (manufactured by Union Showa, Pellet 3A). The obtained fibrous formed article had an average fiber diameter of 4.1 $\mu$m and a thickness of 97 $\mu$m. The number of carbon atoms C (at%) and the number of oxygen atoms O (at%) in the fiber surface were 73.7 and 23.5, respectively. The amount of dioleoylphosphatidylethanolamine in the fiber surface was 67.7 wt%. The surface segregation degree of amphiphilic molecules is 13.5.

[Example 8]

A fibrous formed article was prepared in the same manner as in Example 1, except that 1.0 part by weight of lyophilized dioleoylphosphatidylethanolamine (manufactured by NOF Corporation) and 9.0 parts by weight of polylactic acid (weight average molecular weight: 262,000, manufactured by Purac) were dissolved in 90 parts by weight of a dichloromethane solution dried over a molecular sieve. The obtained fibrous formed article had an average fiber diameter of 4.4 $\mu$m and a thickness of 101 $\mu$m. The number of carbon atoms C (at%) and the number of oxygen atoms O (at%) in the fiber surface were 76.5 and 20.4, respectively. The amount of dioleoylphosphatidylethanolamine in the fiber surface was 80.9 wt%. The surface segregation degree of amphiphilic molecules is 8.09.

[Example 9]

A fibrous formed article was prepared in the same manner as in Example 1, except that dioleoylphosphatidylethanolamine (manufactured by NOF Corporation) was replaced with dilauroylphosphatidylethanolamine (manufactured by NOF Corporation). The obtained fibrous formed article had an average fiber diameter of 4.6 $\mu$m and a thickness of 109 $\mu$m. The number of carbon atoms C (at%) and the number of oxygen atoms O (at%) in the fiber surface were 66.4 and 32.1, respectively. The amount of dilauroylphosphatidylethanolamine in the fiber surface was 43.2 wt%. The surface segregation degree of amphiphilic molecules is 43.2.

[Example 10]

A fibrous formed article was prepared in the same manner as in Example 1, except that dioleoylphosphatidylethanolamine (manufactured by NOF Corporation) was replaced with dierucoylphosphatidylcholine (manufactured by NOF Corporation). The obtained fibrous formed article had an average fiber diameter of 3.7 $\mu$m and a thickness of 93 $\mu$m. The number of carbon atoms C (at%) and the number of oxygen atoms O (at%) in the fiber surface were 68.0 and 31.2, respectively. The amount of dierucoylphosphatidylcholine in the fiber surface was 33.0 wt%. The surface segregation degree of amphiphilic molecules is 33.0.

[Example 11]

A fibrous formed article was prepared in the same manner as in Example 1, except that dioleoylphosphatidylethanolamine (manufactured by NOF Corporation) was replaced with distearoylphosphatidylcholine (manufactured by NOF Corporation). The obtained fibrous formed article had an average fiber diameter of 4.6 $\mu$m and a thickness of 103 $\mu$m. The number of carbon atoms C (at%) and the number of oxygen atoms O (at%) in the fiber surface were 64.4 and 35.2, respectively. The amount of distearoylphosphatidylcholine in the fiber surface was 20.0 wt%. The surface segregation degree of amphiphilic molecules is 20.0.

[Example 12]

A fibrous formed article was prepared in the same manner as in Example 1, except that dioleoylphosphatidylethanolamine (manufactured by NOF Corporation) was replaced with a nonionic surfactant SPAN 80 (a sorbitan fatty acid ester, manufactured by Tokyo Chemical Industry). The obtained fibrous formed article had an average fiber diameter of 3.9 $\mu$m and a thickness of 98 $\mu$m. The number of carbon atoms C (at%) and the number of oxygen atoms O (at%) in the fiber surface were 62.3 and 37.7, respectively. The amount of SPAN 80 in the fiber surface was 12.4 wt%. The surface segregation degree of amphiphilic molecules is 12.4.

[Example 13]

The fiber formed bodies obtained in Examples 1, 2, 7 to 12 and Comparative Examples 1 and 2 were subjected to a hydrophilicity test. The results are shown in the following table.

| Fibrous formed article Used | Hydrophilicity Test (cm) |
| --- | --- |
| Example 1 | 8.6 |
| Example 2 | 4.1 |
| Example 7 | 4.0 |
| Example 8 | 3.2 |
| Example 9 | 5.8 |
| Example 10 | 8.1 |
| Example 11 | 9.5 |
| Example 12 | 21.8 |
| Comparative Example 1 | 29.6 |
| Comparative Example 2 | 29.2 |

Industrial Applicability

The fibrous formed article of the invention has excellent hydrophilicity and thus is used in medical applications. Examples thereof include materials for the protection of the surface of organs or wound sites, covering materials, sealing materials, artificial dura mater, adhesion barriers, and hemostatic materials.

**Claims**

1.  A fibrous formed article comprising a hydrophobic polymer and amphiphilic molecules,
    wherein the fibrous formed article has an average fiber diameter of 0.05 to 50 $\mu$m, and
    the amphiphilic molecules segregate to a fiber surface.

2.  The fibrous formed article according to claim 1, wherein the surface segregation degree of the amphiphilic molecules is 5.0 or more.

3.  The fibrous formed article according to claim 1 or 2, wherein the amphiphilic molecules are contained in an amount of 0.01 to 20 parts by weight per 100 parts by weight of the fibrous formed article.

4.  The fibrous formed article according to any one of claims 1 to 3, wherein the hydrophobic polymer is at least one member selected from the group consisting of polycarbonate, polystyrene, polyarylate, and aliphatic polyesters.

5.  The fibrous formed article according to any one of claims 1 to 3, wherein the hydrophobic polymer is at least one member selected from the group consisting of polyglycolic acid, polylactic acid, polycaprolactone, and copolymers thereof.

6.  The fibrous formed article according to any one of claims 1 to 5, wherein the amphiphilic molecules are at least one member selected from the group consisting of phospholipids, sorbitan fatty acid esters, glycolipids, steroids, and polyamino acids.

7.  The fibrous formed article according to any one of claims 1 to 5, wherein the amphiphilic molecules are phosphatidylcholine and/or phosphatidylethanolamine.

8.  The fibrous formed article according to any one of claims 1 to 7, produced by electrostatic spinning.

<table>
<tr><td colspan="2" align="center">**INTERNATIONAL SEARCH REPORT**</td><td>International application No.<br>PCT/JP2010/070415</td></tr>
</table>

A. CLASSIFICATION OF SUBJECT MATTER
*D04H3/00*(2006.01)i, *A61L15/16*(2006.01)i, *A61L27/00*(2006.01)i, *D01D5/04*
(2006.01)i, *D01F6/00*(2006.01)i, *D04H1/40*(2006.01)i, *D04H1/72*(2006.01)i

According to International Patent Classification (IPC) or to both national classification and IPC

B. FIELDS SEARCHED

Minimum documentation searched (classification system followed by classification symbols)
D04H3/00, A61L15/16, A61L27/00, D01D5/04, D01F6/00, D04H1/40, D04H1/72

Documentation searched other than minimum documentation to the extent that such documents are included in the fields searched
| | | | |
|---|---|---|---|
| Jitsuyo Shinan Koho | 1922-1996 | Jitsuyo Shinan Toroku Koho | 1996-2011 |
| Kokai Jitsuyo Shinan Koho | 1971-2011 | Toroku Jitsuyo Shinan Koho | 1994-2011 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X | WO 2006/022430 A1 (Teijin Ltd.),<br>02 March 2006 (02.03.2006),<br>claims; page 8; examples<br>& JP 4354996 B2 | 1-8 |
| A | WO 2004/072336 A1 (Teijin Ltd.),<br>26 August 2004 (26.08.2004),<br>claims; examples<br>& EP 1600533 A1          & US 2006/0204750 A1 | 1-8 |
| A | JP 2009-207430 A (Asahi Kasei Corp.),<br>17 September 2009 (17.09.2009),<br>claims; drawings; examples<br>(Family: none) | 1-8 |

☐ Further documents are listed in the continuation of Box C.      ☐ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search<br>16 February, 2011 (16.02.11) | Date of mailing of the international search report<br>01 March, 2011 (01.03.11) |
|---|---|
| Name and mailing address of the ISA/<br>Japanese Patent Office | Authorized officer |
| Facsimile No. | Telephone No. |

Form PCT/ISA/210 (second sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2004072336 A

- WO 2006022430 A

**Non-patent literature cited in the description**

- **K. KIM ; M. YU.** *Biomaterials,* 2003, vol. 24, 4977

- **N. SAITO ; T. OKADA.** *Nat. Biotech.,* 2001, vol. 19, 332